# EUROPEAN PATENT APPLICATION

(11) **EP 4 748 302 A1**
(43) Date of publication of application: **27.05.2026**
(21) Application number: 24842334.5
(22) Date of filing: 13.07.2024
(51) Int. Cl.: A61B 5/022

(54) **BLOOD PRESSURE MEASUREMENT METHOD AND SYSTEM**

(30) Priority: 18.07.2023 CN 202310886407; 12.07.2024 CN 202410934518
(71) Applicant: Andon Health Co., Ltd., Tianjin 300190 (CN)
(72) Inventor: LI, Qin, Tianjin 300190 (CN); CONG, Ming, Tianjin 300190 (CN); ZHANG, Ruijun, Tianjin 300190 (CN)
(74) Representative: Santarelli
(86) International application number: PCT/CN2024/105374
(87) International publication number: WO 2025/016341

(57) **Abstract**

A blood pressure measurement method and system are provided. The measurement method includes: incrementally applying a pressure to a user limb to occlude a target artery; obtaining, during pressure application to the user limb, at least a plurality of pressure oscillation wave signals generated by pulsations of the target artery; obtaining a first target signal based on the plurality of pressure oscillation wave signals, and obtaining a diastolic blood pressure based on the first target signal; decrementally applying the pressure to the user limb; detecting a second target signal during decremental pressure application to the user limb, and obtaining the pressure applied to the user limb when the second target signal is detected, where the second target signal is used to represent a state transition of the target artery from an occluded state to a perfused state; and obtaining a systolic blood pressure based on at least the pressure applied to the user limb when the second target signal is detected.

## Description

### Technical Field

The present disclosure relates to the technical field of electronic sphygmomanometers, and in particular, to a blood pressure measurement method and system.

### Background of the Invention

Blood pressure is one of the vital physiological indicators of the human body and serves as a key physiological parameter reflecting cardiovascular function. Common non-invasive blood pressure measurement apparatuses include: mercury sphygmomanometers, which utilize the Korotkoff sound-based auscultatory method, and electronic sphygmomanometers, which employ the oscillometric method. The principles of the above two blood pressure measurement techniques are as follows:
1) Korotkoff sound-based auscultatory method: An inflatable cuff is used to compress the artery in the limb. As the cuff pressure decreases, the artery gradually transitions from complete occlusion to full perfusion. A stethoscope is used to detect sound changes in arterial blood flow during the transition from occlusion to perfusion. A systolic blood pressure and a diastolic blood pressure are determined by referencing the corresponding gas pressure in the cuff.
2) Oscillometric method: An inflatable cuff compresses the artery in the limb to obstruct arterial blood flow, and pressure oscillation waves within the inflatable cuff are detected and collected during inflation or deflation. A systolic blood pressure and a diastolic blood pressure are then calculated based on the pressure oscillation waves.

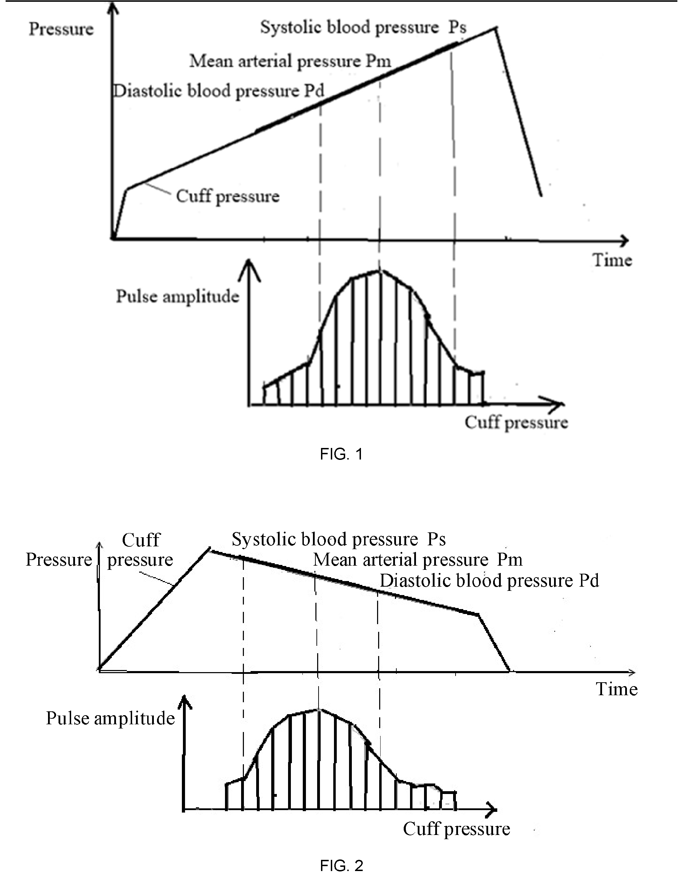

FIG. 1 is a schematic diagram of variation of an amplitude of the pulse (pressure oscillation wave signal) with the inflatable cuff pressure and a schematic diagram of variation of the corresponding cuff pressure (inflatable cuff pressure) over time during pressure increase. As the cuff pressure slowly increases from 0 mmHg, the amplitude of the pulse gradually rises. When the cuff pressure equals the mean arterial pressure Pm, the arterial wall is in an unloaded state, and the amplitude reaches the maximum. After the cuff pressure exceeds the mean arterial pressure Pm, the amplitude gradually decreases. When the cuff pressure further increases beyond the systolic blood pressure Ps, the pressure oscillation wave signals obtained are sufficient for calculating the systolic blood pressure using the oscillometric method. The cuff can be rapidly deflated to conclude the measurement process. During increase of the cuff pressure, the amplitudes of the pulse oscillation waves are recorded, forming an approximately parabolic envelope. Based on statistical data from large populations, different manufacturers can identify envelope curves of the systolic blood pressure Ps and diastolic blood pressure Pd, as well as curve characteristics of the pulse amplitude for various blood pressure types, and construct corresponding statistical models. The algorithms related to these statistical models are embedded in the memory of electronic sphygmomanometers and applied during individual user measurements to calculate the systolic blood pressure and diastolic blood pressure.

FIG. 2 is a schematic diagram of variation of the amplitude of the pulse (pressure oscillation wave signal) with the inflatable cuff pressure and a schematic diagram of variation of the corresponding cuff pressure (inflatable cuff pressure) over time during pressure reduction.

First, the cuff is rapidly inflated. When the cuff pressure exceeds the systolic blood pressure Ps, arterial blood flow is blocked, and small oscillation waves appear in the cuff due to proximal pulse impacts. When the cuff begins to deflate slowly, pressure oscillation wave signals are collected. When the cuff pressure falls below the systolic blood pressure Ps, the amplitude of the pulse wave gradually increases. When the cuff pressure equals the mean arterial pressure Pm, the arterial wall is in an unloaded state, and the amplitude of the pulse wave reaches the maximum. After the cuff pressure drops below the mean arterial pressure, the amplitude of the pulse wave gradually decreases. Once the cuff slowly deflates to below the diastolic blood pressure Pd, the oscillation wave signals are not collected and the cuff can be rapidly deflated to end the measurement. During gradual decrease of the cuff pressure, the amplitudes of the collected pressure oscillation waves of the pulse form an approximately parabolic envelope. Based on statistical data from large populations, different manufacturers can identify envelope curves of the systolic blood pressure Ps and diastolic blood pressure Pd, as well as curve characteristics of the pulse amplitude for various blood pressure types, and construct corresponding statistical models. The algorithms related to these statistical models are embedded in the memory of electronic sphygmomanometers and applied during individual user measurements to calculate the systolic blood pressure and diastolic blood pressure.

The above blood pressure measurement principles have the following disadvantages:
1. The mercury sphygmomanometer using the Korotkoff sound-based auscultatory method is the gold standard for blood pressure measurement. However, with the global enforcement of the Minamata Convention on Mercury on 16 August 2017, mercury-containing blood pressure measurement apparatuses are to be gradually phased out.
   To ensure measurement accuracy for the systolic blood pressure and diastolic blood pressure, the cuff deflation rate needs to be controlled to at least 5 mmHg per second or per pulse. For higher measurement precision, the deflation rate may even need to be reduced to 2-3 mmHg per second or per pulse, resulting in prolonged measurement time and reduced comfort.
2. The electronic sphygmomanometers using the oscillometric method rely on unified models obtained through large-scale population statistics. For certain specific populations, individual adaptability is poor, and measurement accuracy is lower than that of the Korotkoff sound-based auscultatory method.
   Moreover, whether using the inflation or deflation oscillometric method to calculate the systolic blood pressure and diastolic blood pressure, a sufficient number of pulse signals need to be collected to plot the envelope curve. Therefore, the inflation or deflation rate during measurement cannot be too fast. Typically, the pressure change rate during measurement is limited to approximately 4-6 mmHg per second or per pulse, with the maximum pressure increase exceeding the systolic blood pressure by about 20-30 mmHg. Additionally, the rapid deflation phase during inflation-based measurement or the rapid inflation phase during deflation-based measurement also requires a certain duration. Consequently, the overall measurement time for electronic sphygmomanometers using the oscillometric method is prolonged. For example, for a user with a systolic blood pressure of 130 mmHg, the entire measurement process may last over 30 seconds. The higher the user's blood pressure, the greater the required pressure increase and the longer the measurement time. For a user with a systolic blood pressure around 200 mmHg, the measurement process may take about one minute, resulting in prolonged arm compression and reduced comfort.
3. Some products simply combine the inflation and deflation measurement processes of the oscillometric method to improve measurement accuracy. However, since the oscillometric principle requires the measurement process to cover sufficient pulse signals throughout the full pressure range from the systolic blood pressure to the diastolic blood pressure, combining the inflation and deflation measurement processes results in excessively long arm compression time and reduced comfort.

### Summary of the Invention

The present disclosure aims to provide a blood pressure measurement method and system to improve the precision of blood pressure measurement.

According to a first aspect, the present disclosure provides a blood pressure measurement method, comprising: incrementally applying a pressure to a user limb to block a target artery; obtaining, during pressure application to the user limb, at least a plurality of pressure oscillation wave signals generated by pulsations of the target artery; obtaining a first target signal based on the plurality of pressure oscillation wave signals, and obtaining a diastolic blood pressure based on the first target signal; decrementally applying the pressure to the user limb; detecting a second target signal during decremental pressure application to the user limb, and obtaining the pressure applied to the user limb when the second target signal is detected, wherein the second target signal is used to represent a state transition of the target artery from an occluded state to a perfused state; and obtaining a systolic blood pressure based on at least the pressure applied to the user limb when the second target signal is detected.

In one possible embodiment, decrementally applying the pressure to the user limb comprises: reducing the pressure at a first average pressure reduction rate, and subsequently reducing the pressure at a second average pressure reduction rate; and detecting the second target signal during pressure reduction comprises: detecting the second target signal during pressure reduction at the first average pressure reduction rate, wherein the second average pressure reduction rate is greater than the first average pressure reduction rate.

In another possible embodiment, optionally in combination with one or more of the embodiments described above, the blood pressure measurement method comprises: switching to reducing the pressure at the second average pressure reduction rate in response to confirming detection of the second target signal.

In another possible embodiment, optionally in combination with one or more of the embodiments described above, incrementally applying the pressure to the user limb comprises: first applying the pressure at a first average pressure increase rate and subsequently applying the pressure at a second average pressure increase rate, wherein the first average pressure increase rate is less than the second average pressure increase rate.

In another possible embodiment, optionally in combination with one or more of the embodiments described above, the blood pressure measurement method comprises: detecting a third target signal during pressure application, and stopping applying the pressure in response to failing to detect the third target signal; or stopping applying the pressure in response to reaching a preset pressure threshold, wherein the preset pressure threshold is set based on the first target signal.

In another possible embodiment, optionally in combination with one or more of the embodiments described above, the third target signal is a fluctuation signal obtained from downstream of the target artery to which the pressure is applied during incremental pressure application to the user limb, the fluctuation signal is an air pressure signal, a photoelectric signal, an electromagnetic wave signal, an ultrasonic signal, or a piezoelectric signal, and the fluctuation signal reflects changes in blood flow.

In another possible embodiment, optionally in combination with one or more of the embodiments described above, the first target signal is a preset pressure oscillation wave signal obtained based on the plurality of pressure oscillation wave signals, and/or the second target signal is a first fluctuation signal detected during decremental pressure application to the user limb, the fluctuation signal is an air pressure signal, a photoelectric signal, an electromagnetic wave signal, an ultrasonic signal, or a piezoelectric signal, and the fluctuation signal reflects changes in blood flow.

In another possible embodiment, optionally in combination with one or more of the embodiments described above, the first fluctuation signal is a first fluctuation signal of at least two consecutive fluctuation signals detected for a first time.

In another possible embodiment, optionally in combination with one or more of the embodiments described above, the blood pressure measurement method further comprises: calculating a predicted systolic blood pressure based on the first target signal, and calculating the systolic blood pressure based on the predicted systolic blood pressure and the pressure applied to the user limb when the second target signal is detected.

In another possible embodiment, optionally in combination with one or more of the embodiments described above, calculating the systolic blood pressure based on at least the second target signal comprises: based on a moment of detecting the second target signal, obtaining the pressure applied to the user limb at the moment.

In another possible embodiment, optionally in combination with one or more of the embodiments described above, the second target signal is detected downstream of a position at which the target artery is occluded.

According to a second aspect, the present disclosure provides a blood pressure measurement system, comprising: a pressure application apparatus configured to apply a pressure to a user limb; a first sensing apparatus configured to obtain pressure signals; a second sensing apparatus configured to obtain a second target signal; and a controller programmed to: control the pressure application apparatus to incrementally apply the pressure to the user limb to block a target artery; control the first sensing apparatus to obtain at least a plurality of pressure oscillation wave signals generated by pulsations of the target artery during pressure application to the user limb; calculate a first target signal based on the plurality of pressure oscillation wave signals, and obtain a diastolic blood pressure based on the first target signal; control the pressure application apparatus to decrementally apply the pressure to the user limb; control the second sensing apparatus to detect a second target signal during decremental pressure application to the user limb, and obtain, from the first sensing apparatus, the pressure applied to the user limb when the second target signal is detected, wherein the second target signal is used to represent a state transition of the target artery in the user limb from an occluded state to a perfused state; and obtain a systolic blood pressure based on at least the pressure applied to the user limb when the second target signal is detected.

In one possible embodiment, the controller is programmed to: control the pressure application apparatus to reduce the pressure at a first average pressure reduction rate, and subsequently reduce the pressure at a second average pressure reduction rate during the decremental pressure application to the user limb; and control the second sensing apparatus to detect, when detecting the second target signal during pressure reduction, the second target signal during pressure reduction at the first average pressure reduction rate, wherein the second average pressure reduction rate is greater than the first average pressure reduction rate.

In another possible embodiment, optionally in combination with one or more of the embodiments described above, the controller is programmed to control the pressure application apparatus to switch to reducing the pressure at the second average pressure reduction rate in response to confirming detection of the second target signal.

In another possible embodiment, optionally in combination with one or more of the embodiments described above, the controller is programmed to: control the pressure application apparatus to apply the pressure at a first average pressure increase rate, and subsequently apply the pressure at a second average pressure increase rate during incremental pressure application to the user limb, wherein the first average pressure increase rate is less than the second average pressure increase rate.

In another possible embodiment, optionally in combination with one or more of the embodiments described above, the controller is programmed to control the second sensing apparatus to detect a third target signal when the pressure application apparatus applies the pressure, and control the pressure application apparatus to:stop applying the pressure in response to failing to detect the third target signal; or stop applying the pressure in response to reaching a preset pressure threshold, wherein the preset pressure threshold is set based on the first target signal.

In another possible embodiment, optionally in combination with one or more of the embodiments described above, the third target signal is a fluctuation signal obtained from downstream of the target artery to which the pressure is applied during incremental pressure application to the user limb, the fluctuation signal is an air pressure signal, a photoelectric signal, an electromagnetic wave signal, an ultrasonic signal, or a piezoelectric signal, and the fluctuation signal reflects changes in blood flow.

In another possible embodiment, optionally in combination with one or more of the embodiments described above, the first target signal is a preset pressure oscillation wave signal obtained based on the plurality of pressure oscillation wave signals, and/or the second target signal is a first fluctuation signal detected during reduction of the pressure applied to the user limb, the fluctuation signal is an air pressure signal, a photoelectric signal, an electromagnetic wave signal, an ultrasonic signal, or a piezoelectric signal, and the fluctuation signal reflects changes in blood flow.

In another possible embodiment, optionally in combination with one or more of the embodiments described above, the first fluctuation signal is a first fluctuation signal of at least two consecutive fluctuation signals detected for a first time.

In another possible embodiment, optionally in combination with one or more of the embodiments described above, the controller further calculates a predicted systolic blood pressure based on the first target signal, and calculates the systolic blood pressure based on the predicted systolic blood pressure and the pressure applied to the user limb when the second target signal is detected.

In another possible embodiment, optionally in combination with one or more of the embodiments described above, a process in which the controller calculates the systolic blood pressure based on at least the second target signal comprises: based on a moment of detecting the second target signal, obtaining the pressure applied to the user limb at the moment.

In another possible embodiment, optionally in combination with one or more of the embodiments described above, the second sensing apparatus is configured to detect the second target signal downstream of a position at which the target artery is occluded.

The blood pressure measurement method provided in the present disclosure utilizes the oscillometric method to obtain signals and calculate the diastolic blood pressure during pressure increase on the limb, and to obtain the systolic blood pressure by detecting a transition signal indicating that the blood vessel switches from occlusion to perfusion during pressure reduction. This method improves the measurement precision of both the systolic blood pressure and the diastolic blood pressure without significantly increasing the measurement time.

### Brief Description of the Drawings

To describe the technical solutions in the embodiments of the present disclosure more clearly, the following briefly describes the accompanying drawings required for describing the embodiments of the present disclosure. Apparently, the accompanying drawings in the following description show merely some embodiments of the present disclosure, and those of ordinary skill in the art may still derive other drawings from these accompanying drawings without creative efforts.
FIG. 1 is a schematic diagram of variation of a pulse amplitude with an inflatable cuff pressure, and a schematic diagram of variation of the cuff pressure over time in blood pressure measurement using an oscillometric method during inflation;
FIG. 2 is a schematic diagram of variation of a pulse amplitude with an inflatable cuff pressure, and a schematic diagram of variation of the cuff pressure over time in blood pressure measurement using an oscillometric method during deflation;
FIG. 3 is a flowchart of a blood pressure measurement method according to an embodiment of the present disclosure;
FIG. 4 is a schematic structural diagram of a blood pressure measurement system according to an embodiment of the present disclosure;
FIG. 5 is another schematic structural diagram of the blood pressure measurement system in FIG. 4;
FIG. 6 is a detailed schematic structural diagram of the blood pressure measurement system in FIG. 5;
FIG. 7 is a schematic diagram of variation of a pressure inside a first inflatable cuff over time when using a blood pressure measurement method according to an embodiment of the present disclosure; and
FIG. 8 is a schematic diagram of variation of a pressure inside a first inflatable cuff over time when using a blood pressure measurement method according to another embodiment of the present disclosure.

### Detailed Description of Embodiments

To make the objectives, technical solutions, and advantages of the embodiments of the present disclosure clearer, the following clearly and completely describes the technical solutions in the embodiments of the present disclosure with reference to the accompanying drawings in the embodiments of the present disclosure. Apparently, the described embodiments are some but not all of the embodiments of the present disclosure. All other embodiments obtained by a person of ordinary skill in the art based on the embodiments of the present disclosure without creative efforts should fall within the protection scope of the present disclosure.

The following provides detailed description of features and exemplary embodiments of various aspects of the present disclosure. In the detailed description below, many specific details are illustrated to provide a comprehensive understanding of the present disclosure. However, it should be appreciated by those of skill in the art that the present disclosure may be implemented without some of these specific details. The following embodiments are described only to provide a better understanding of the present disclosure by illustrating examples of the present disclosure. The present disclosure is not limited to any specific structures and configurations presented below, but covers any modifications, replacements, and improvements of parts, components, and connection methods without departing from the spirit of the present disclosure. In the accompanying drawings and the description below, well-known structures and techniques are not shown in order to avoid unnecessary obscurity in the present disclosure.

The described features, structures, or characteristics may be incorporated into one or more embodiments in any suitable manner. In the following description, many specific details are provided to give a full understanding of the embodiments of the present disclosure.

A blood pressure measurement method provided in the present disclosure includes obtaining a diastolic blood pressure in a process of increasing a pressure to a user limb and obtaining a systolic blood pressure based on at least a signal obtained in a process of reducing the pressure applied to the user limb. This improves the precision of blood pressure measurement. The main procedure of the method includes: A pressure application apparatus, such as a cuff bladder and an air pump, incrementally applies a pressure to the user limb to occlude a target artery; a pressure sensor obtains a plurality of pressure oscillation wave signals generated by pulsations of the target artery during application of the pressure to the user limb; and a first target signal (such as a pressure oscillation wave signal with a maximum pulse amplitude detected during blood pressure measurement using an oscillometric method) is obtained based on the plurality of pressure oscillation wave signals, and the diastolic blood pressure is obtained based on the first target signal. Subsequently, the pressure applied to the user limb is gradually reduced, and a second target signal and a pressure applied to the user limb when the second target signal is detected are detected during the pressure reduction process. The second target signal is used to represent a state transition of the target artery from occlusion to perfusion. The systolic blood pressure is obtained based on at least the pressure applied to the user limb when the second target signal is detected.

This measurement method fully leverages the characteristics of pressure increase during incremental pressure increase to the limb. For example, during an initial stage of pressure increase, the pressure in the inflatable cuff increases slowly, allowing more pressure oscillation wave data to be obtained at lower pressure. This results in an ideal envelope of the pressure oscillation wave signals, providing a more accurate diastolic blood pressure. Additionally, the systolic blood pressure is obtained based on at least the second target signal, which represents the state transition of the target artery from occlusion to perfusion. This is more adaptable to individual differences compared to traditional oscillometric methods that calculate the systolic blood pressure using the amplitude coefficient method after obtaining the mean arterial pressure. Therefore, the systolic blood pressure obtained is more accurate.

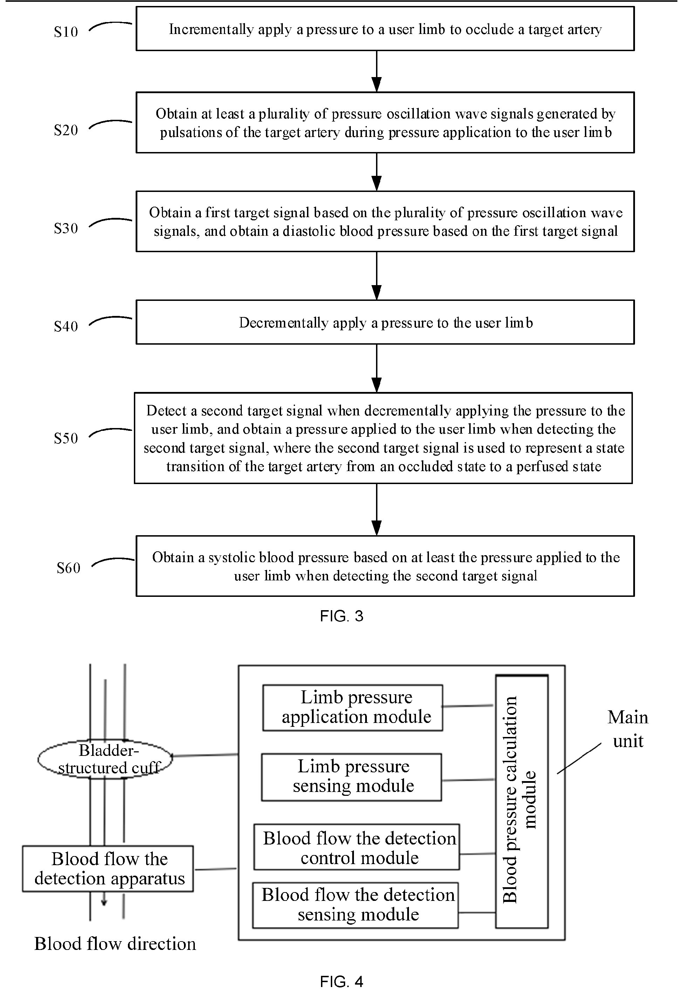

FIG. 3 is a flowchart of a blood pressure measurement method according to an embodiment of the present disclosure.

In this embodiment, the blood pressure measurement method includes the following steps:
In S10, a pressure is incrementally applied to a user limb to occlude a target artery.

Specifically, an inflatable cuff may be used to wrap around an upper arm of a user, and is inflated using an air pump. During the inflation process, the pressure in the inflatable cuff gradually increases at an average pressure increase rate V1. As the inflatable cuff gradually expands, the brachial artery is compressed. When sufficiently inflated, the inflatable cuff can occlude the brachial artery.

In S20, at least a plurality of pressure oscillation wave signals generated by pulsations of the target artery during pressure application to the user limb are obtained.

The pressure inside the inflatable cuff can be considered as the pressure applied to the brachial artery. Furthermore, due to the force exerted by arterial pulsations on the inflatable cuff, pressure oscillation waves corresponding to the pulse are generated in the inflatable cuff. The inflatable cuff may be connected via a tube to a pressure sensor. The pressure sensor can read real-time pressure signals in the inflatable cuff during pressure application to the user limb. Static pressure signals and pressure oscillation wave signals corresponding to the pulse can be extracted from the real-time pressure signals. The static pressure signal refers to the pressure signal smoothly increasing or decreasing over time as the inflatable cuff is inflated or deflated.

In S30, a first target signal is obtained based on the plurality of pressure oscillation wave signals, and a diastolic blood pressure is obtained based on the first target signal.

The static pressure signal is the pressure signal smoothly varying over time within the inflatable cuff and can be considered as the pressure applied to the brachial artery. The pressure oscillation wave signal represents pulsations of the brachial artery. The oscillation wave amplitude of the pressure oscillation wave signal exhibits the following pattern: During pressure increase, before the pressure within the inflatable cuff reaches a mean arterial pressure Pm, the oscillation wave amplitude gradually increases with the increasing pressure; when the pressure within the inflatable cuff equals the mean arterial pressure Pm, the arterial wall is in an unloaded state and the oscillation wave amplitude reaches the maximum; after the pressure within the inflatable cuff surpasses the mean arterial pressure Pm, the oscillation wave amplitude gradually decreases with the increasing pressure. During the gradual increase of the pressure within the inflatable cuff, the amplitudes of the plurality of pressure oscillation wave signals form an approximately parabolic envelope. A pressure oscillation wave signal with the largest amplitude is the first target signal.

After obtaining the first target signal, an amplitude of a pressure oscillation wave signal corresponding to the diastolic blood pressure can be further calculated based on a preset amplitude coefficient. However, the calculated amplitude may not perfectly match a pressure oscillation wave signal. The first pressure oscillation wave signal with an amplitude slightly less than the calculated amplitude may be selected to estimate the diastolic blood pressure, and a static pressure signal within the inflatable cuff corresponding to a starting point of the pressure oscillation wave signal is used as the diastolic blood pressure. Alternatively, the diastolic blood pressure can be estimated using two adjacent pressure oscillation wave signals whose amplitudes are adjacent to the amplitude of the pressure oscillation wave signal corresponding to the diastolic blood pressure. The proportional relationship between the amplitude corresponding to the diastolic blood pressure and the amplitudes of the two adjacent pressure oscillation wave signals is calculated, and the proportional relationship is used to interpolate between static pressure values of the inflatable cuff corresponding to starting points of the two pressure oscillation wave signals, to calculate the value of diastolic blood pressure.

In S40, a pressure is decrementally applied to the user limb.

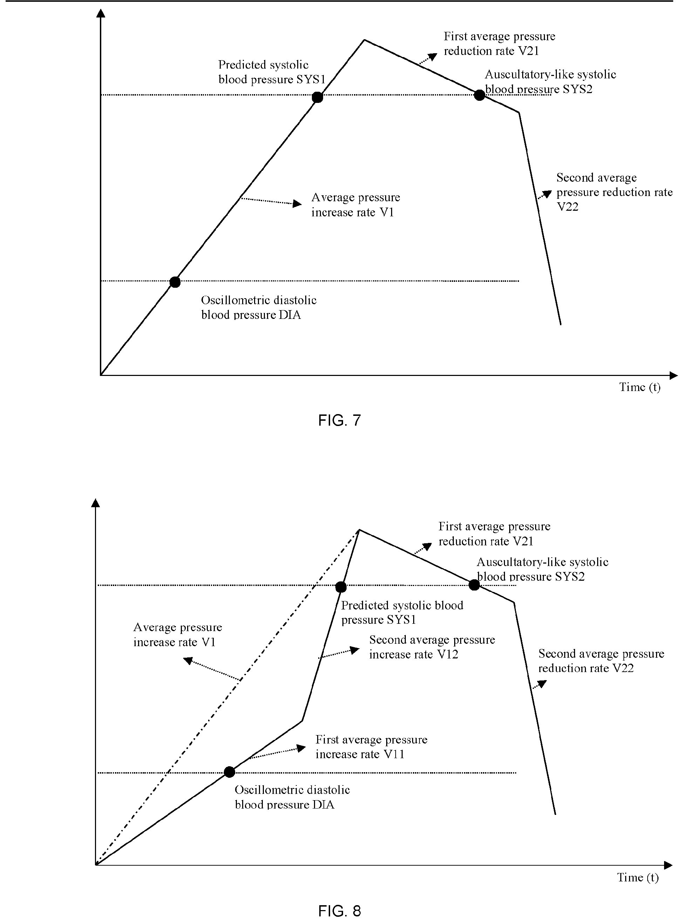

The inflatable cuff can be deflated using a valve with a controllable exhaust rate, to gradually reduce the air pressure within the inflatable cuff, such that the pressure on the brachial artery gradually decreases until blood flow resumes within the artery. As shown in FIG. 7, the pressure inside the inflatable cuff is reduced at a first average pressure reduction rate V11.

In S50, a second target signal is detected during the pressure reduction process, and a pressure applied to the user limb when the signal is detected is obtained. The second target signal is used to represent a state transition of the target artery from an occluded state to a perfused state.

When the pressure on the brachial artery decreases from a value greater than the systolic blood pressure to a value equal to the systolic blood pressure, the blood flow within the artery transitions from the occluded state to a reopened state. Pulsations reappear downstream of a previously occluded position on the artery. Therefore, the pressure oscillation wave signal can also be detected downstream. Specifically, the pressure oscillation wave signal can be detected at the artery downstream of the inflatable cuff using a small cuff bladder and a pressure sensor or another detection apparatus. The first detected pressure oscillation wave signal is the second target signal. Alternatively, Korotkoff sounds are detected at the artery downstream of the inflatable cuff, and a Korotkoff sound signal is used as the second target signal.

In S60, the systolic blood pressure is obtained based on at least the pressure (auscultatory-like systolic blood pressure SYS2) applied to the user limb when the signal is detected.

At a moment when blood flow resumes in the brachial artery, the pressure inside the blood vessel equals the pressure in the inflatable cuff. The state transition of the blood flow in the brachial artery and a moment corresponding to the transition are detected, to obtain the static pressure within the inflatable cuff at this moment. The static pressure is the systolic blood pressure.

The method employed by electronic sphygmomanometers using the auscultatory-like pulse wave method is similar to the Korotkoff sound-based auscultatory method. During use, a sensor for detecting pulse waves needs to be placed at a location downstream along the blood flow direction relative to the inflatable cuff used for generating Korotkoff sounds. After the inflatable cuff is rapidly inflated to a pressure 20-30 mmHg higher than a systolic blood pressure of a subject, slow deflation begins. During the pressure reduction process, a distinct pulse oscillation signal is generated at a moment when the artery transitions from being completely collapsed to being forced open by blood flow, and this change in the blood flow is detected by the downstream pulse wave sensor. The pressure within the inflatable cuff corresponding to this moment is the systolic blood pressure. Subsequently, the inflatable cuff continues to slowly deflate, and the pulse wave sensor stably detects pulse signals. When the artery is fully perfused and blood flows normally, the pressure within the inflatable cuff is the diastolic blood pressure. After detecting the diastolic blood pressure, rapid deflation can be initiated to end the measurement. However, during the transition from the reopening of blood flow to the artery being fully perfused, changes in the pulse signals are not significant, leading to larger errors in calculation of the diastolic blood pressure. Furthermore, to ensure measurement precision when using the auscultatory-like method for calculating the systolic blood pressure and diastolic blood pressure, the deflation rate needs to be limited to 5 mmHg per second or per pulse, or even controlled to 2-3 mmHg per second or per pulse. Combined with the pressure increase phase, the entire measurement process subjects the arm to prolonged compression, resulting in poor comfort.

During inflation of the inflatable cuff, the air pump generally outputs pressurized gas at a constant rate. In the initial phase of inflation, as the internal air volume increases, the inflatable cuff expands, causing the pressure within the inflatable cuff to increase slowly. During the later phase of expansion of the inflatable cuff, the volume no longer increases significantly as more air is added, leading to a rapid pressure increase. Compared to the rapid pressure increase phase of the inflatable cuff, the earlier slow pressure increase phase lasts longer, allowing collection of more pressure oscillation wave signals during this period. This facilitates the generation of more ideal envelope curves of the pressure oscillation wave signals and the amplitudes, thereby yielding a more accurate diastolic blood pressure. Moreover, during the deflation phase of the inflatable cuff in which the pressure on the user limb is reduced, only the second target signal generated at the moment when the target artery transits from the occluded state to the perfused state is obtained, to obtain the systolic blood pressure. Once the systolic blood pressure is obtained, the systolic blood pressure and diastolic blood pressure can be output. Compared to obtaining the systolic blood pressure using the mean arterial pressure Pm and the amplitude coefficient obtained based on statistics, the method in the present disclosure, which obtains the systolic blood pressure using the auscultatory-like method, offers better adaptability to different user types and higher individual precision. The measurement method of the present disclosure takes less time compared to the traditional oscillometric method that simply combines the pressure increase measurement process and pressure reduction measurement process. The latter leads to excessively long measurement time with the user limb under pressure, resulting in poor comfort.

If the pressure increase measurement process of the oscillometric method is simply combined with the pressure reduction measurement process of the auscultatory-like pulse wave method to improve accuracy, the overall blood pressure measurement time is also prolonged, causing the user arm to be compressed for a long period, easily leading to sensations of numbness, swelling, or even pain.

Compared with the prior art, this embodiment of the present disclosure has at least the following beneficial effects:
1. Improved measurement accuracy of the diastolic blood pressure and systolic blood pressure:
   The present disclosure employs a method combining the auscultatory-like method and the oscillometric method. During application of pressure to the limb, sufficient pulse signals in the low-pressure phase of the oscillometric method are fully utilized to calculate the diastolic blood pressure only, ensuring the measurement accuracy of the diastolic blood pressure. In addition, when reducing the pressure on the limb, the principle of the auscultatory-like pulse wave method is adopted. Compared to the principle of the statistics-based oscillometric method, the auscultatory-like method completely reflects the individual state when blood flow forces the artery open, offering better individual adaptability and higher accuracy for the systolic blood pressure.
2. Reduced non-invasive blood pressure measurement time:
   The method of the present disclosure allows for faster pressure increase rate in the high-pressure phase. Since the systolic blood pressure does not need to be calculated during the pressure increase measurement phase, the pressure increase rate in the high-pressure phase can be increased to above 10 mmHg/s, resulting in shorter pressure increase time.

In the present disclosure, a cuff pressure increase is predicted without prior knowledge of the systolic blood pressure, ensuring that the maximum pressure increase is only slightly higher than the systolic blood pressure of the subject. The pressure is increased to the required pressure based on different blood pressure levels, thereby reducing unnecessary arm compression time.

In the pressure reduction process of the present disclosure, rapid deflation is performed once the systolic blood pressure is calculated, thereby ending the measurement. Since the diastolic blood pressure is already known from the pressure increase process, there is no need for repeated calculation in the pressure reduction process, significantly shortening the measurement time. For example, the systolic blood pressure is 130 mmHg and the diastolic blood pressure is 80 mmHg. Compared to the oscillometric method and the auscultatory-like method requiring pressure reduction below 80 mmHg in the pressure reduction measurement process, approximately 50 mmHg of slow pressure reduction is eliminated, saving about 10 s for pressure reduction. For hypertensive patients with a large systolic-diastolic blood pressure difference, the saved measurement time is more significant. For a subject with the systolic/diastolic blood pressure of 180/80 mmHg, the pressure reduction process alone saves approximately 20 s in the present disclosure.

3. Enhanced comfort for the subject during the entire blood pressure measurement process:
Compared to existing blood pressure measurement principles, the present disclosure significantly reduces the overall measurement time and shortens the duration of limb compression, leading to better comfort. Furthermore, by estimating the systolic blood pressure of the subject to predict the required pressure increase, the present disclosure avoids applying excessively high pressure on the limb. This effectively reduces sensations of numbness, swelling, or even pain in the arm during measurement, thereby improving the comfort of blood pressure measurement.

In some embodiments, incrementally applying the pressure to the user limb to occlude the target artery in step S10 specifically includes: first applying the pressure at a first average pressure increase rate V11, and subsequently applying the pressure at a second average pressure increase rate V12. The first average pressure increase rate V11 is less than the second average pressure increase rate V12. FIG. 8 is a schematic diagram of variation of the pressure inside the inflatable cuff over time when the pressure is applied to the user limb at the first average pressure increase rate V11 and the second average pressure increase rate V12. The second average pressure increase rate V12 is greater than the first average pressure increase rate V11. During pressure increase at the first average pressure increase rate, the pressure increase inside the inflatable cuff may be non-linear (for example, a pressure increase curve exhibits a parabolic shape). During pressure increase at the second average pressure increase rate, the pressure increase may also be non-linear (for example, the pressure increase curve exhibits a parabolic shape). The overall pressure increase curve for pressure increase at the first and second average pressure increase rates may be two adjacent segments of a parabolic curve. For instance, the pressure increase curve corresponding to the first average pressure increase rate may be a flatter segment of the parabolic curve (a segment with a slower rate of slope increase), while the pressure increase curve corresponding to the second average pressure increase rate may be a steeper segment of the parabolic curve (a segment with a faster rate of slope increase).

In some optional embodiments, the second average pressure increase rate is switched to for pressure application in response to the pressure increasing to a preset pressure at the first average pressure increase rate. For example, after the mean arterial pressure Pm is calculated based on the first target signal, a pressure threshold for switching the pressure increase rate is set. The pressure threshold may be set to a value greater than Pm by the preset value. When the pressure inside the inflatable cuff reaches the pressure threshold, the pressure increase rate is increased.

Furthermore, in some embodiments, in step S10, a third target signal is further detected during pressure application to the user limb, and the pressure application is stopped in response to failing to detect the third target signal. The third target signal is a fluctuation signal obtained during the incremental application of the pressure to the user limb. The fluctuation signal represents blood flow changes at a detection site caused by the pulse, such as a pressure oscillation wave signal of the air pressure. When no fluctuation signal is detected, it indicates that the target artery has been occluded, then the pressure increase is stopped and the pressure reduction process is initiated. For example, a second inflatable cuff paired with a pressure sensor may be provided downstream of the inflatable cuff to detect the pressure oscillation wave signal of the air pressure. In some optional embodiments, the third target signal may alternatively be a fluctuation signal of another type, such as a photoelectric signal, an electromagnetic wave signal, an ultrasonic signal, or a piezoelectric signal. Specifically, during the incremental application of the pressure to the user limb, the fluctuation signal may be obtained downstream of the target artery using a photoelectric sensing apparatus, a bladder pulse sensing apparatus, a radar sensing apparatus, a Doppler sensing apparatus, or a piezoelectric pulse sensing apparatus.

In other embodiments, in step S10, the pressure increase is stopped in response to reaching the preset pressure threshold. The preset pressure threshold is set based on the first target signal. As shown in FIG. 7 and FIG. 8, FIG. 7 is a schematic diagram of variation of the pressure inside the inflatable cuff over time when using the blood pressure measurement method according to an embodiment of the present disclosure. A predicted systolic blood pressure SYS1 may be calculated based on the first target signal and set the pressure threshold based on the predicted systolic blood pressure SYS1. When the pressure inside the inflatable cuff reaches or exceeds the pressure threshold, the pressure increase can be stopped and the pressure reduction process is initiated. Specifically, the first target signal is a pressure oscillation wave signal with the largest amplitude obtained in the process of increasing the pressure to the limb. The corresponding oscillometric diastolic blood pressure DIA is obtained, then the amplitude coefficient is used to obtain the predicted systolic blood pressure SYS1, and finally the pressure threshold is set to be slightly higher than the predicted systolic blood pressure SYS1.

In some embodiments, reducing the pressure applied to the user limb in step S40 specifically includes: reducing the pressure at a first average pressure reduction rate and subsequently reducing the pressure at a second average pressure reduction rate. The second average pressure reduction rate is greater than the first average pressure reduction rate. This allows the inflatable cuff to deflate rapidly, reducing the time for the user limb under pressure. Further, in some embodiments, in step S50, a second target signal is detected when reducing the pressure at the first average pressure reduction rate. Further, in some embodiments, in step S50, the second average pressure reduction rate is switched to for pressure reduction in response to confirming detection of the second target signal. Once the second target signal is detected, the systolic blood pressure of the user can be calculated. Therefore, upon detecting the second target signal, the inflatable cuff begins to deflate at the second average pressure reduction rate to relieve pressure from the user limb as quickly as possible.

In some embodiments, in step S50, the second target signal is a first fluctuation signal detected during the process of reducing the pressure applied to the user limb. The fluctuation signal represents blood flow changes at a detection site caused by the pulse, such as a pressure oscillation wave signal of the air pressure. The pressure oscillation wave signal from the target artery is detected downstream of the occlusion site of the target artery using the second inflatable cuff and the pressure sensor. During reduction of the pressure applied to the user limb, after the artery transitions from an occluded state to a perfused state, the pressure oscillation wave can propagate downstream. A moment at which the first pressure oscillation wave signal is detected can be regarded as the moment at which the artery transitions from the occluded state to the perfused state. Specifically, the starting point of the first pressure oscillation wave signal is used as the moment at which the artery transitions from the occluded state to the perfused state, and subsequently in step S60, the static pressure of the inflatable cuff at this moment can be obtained as the systolic blood pressure. The second target signal may alternatively be a fluctuation signal of another type, such as a photoelectric signal, an electromagnetic wave signal, an ultrasonic signal, or a piezoelectric signal. Specifically, during the decremental application of the pressure to the user limb, the fluctuation signal may be obtained downstream of the target artery using a photoelectric sensing apparatus, a bladder pulse sensing apparatus, a radar sensing apparatus, a Doppler sensing apparatus, or a piezoelectric pulse sensing apparatus.

Further, in some embodiments, in step S50, the first fluctuation signal is the first fluctuation signal in two consecutive fluctuation signals detected for the first time. Specifically, when the cuff bladder is used to collect the fluctuation signal, the fluctuation signal is an air pressure signal, specifically, a pressure oscillation wave signal. The first pressure oscillation wave signal is the first of two consecutive pressure oscillation wave signals detected for the first time. To avoid interference, it can be determined based on the heart rate of the user whether the two detected pressure oscillation wave signals are consecutive. In some optional embodiments, the first fluctuation signal is the first of three consecutive fluctuation signals detected for the first time.

In some optional embodiments, in step S50, the second target signal may be alternatively detected downstream of the target artery using a photoelectric sensing apparatus, a radar sensing apparatus, a Doppler sensing apparatus, or a piezoelectric pulse sensing apparatus.

In some embodiments, in step S60, the predicted systolic blood pressure SYS1 is further calculated based on the first target signal, and the systolic blood pressure is calculated based on both the predicted systolic blood pressure and the pressure (the auscultatory-like systolic blood pressure SYS2) applied to the user limb at a moment when the second target signal is detected. The step of calculating the predicted systolic blood pressure based on the first target signal may include: calculating the systolic blood pressure based on the mean arterial pressure Pm using the amplitude coefficient method, and using the systolic blood pressure as the predicted systolic blood pressure. The step of calculating the systolic blood pressure based on both the predicted systolic blood pressure and the pressure applied to the user limb at a moment when the second target signal is detected may include calculating an average or a weighted average of the two values.

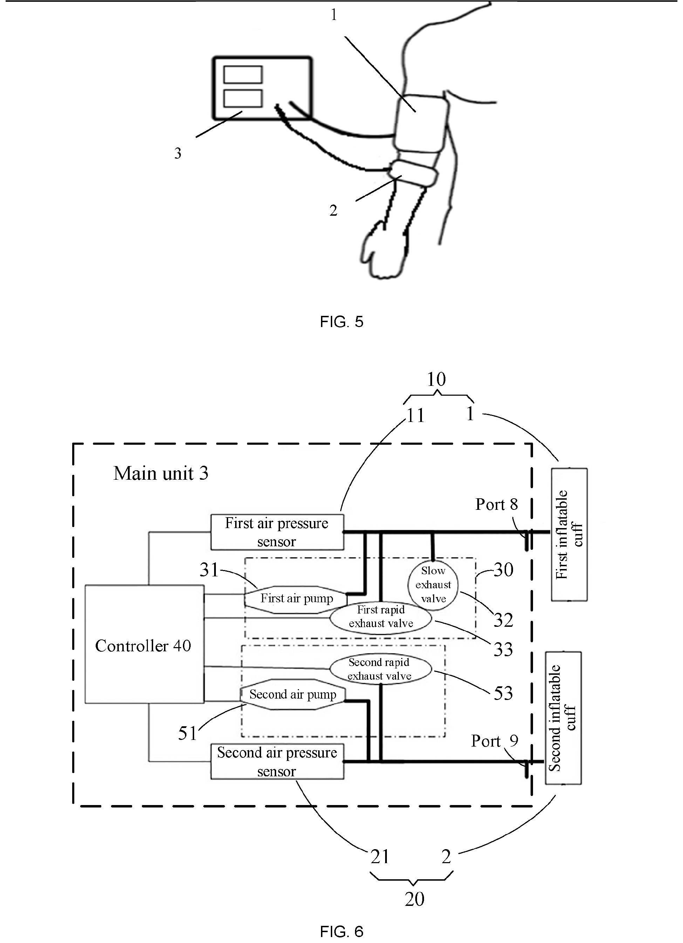

Referring to FIG. 4 to FIG. 6, the present disclosure provides a blood pressure measurement system.

The blood pressure measurement system includes a main unit, a bladder-structured cuff, and a blood flow detection apparatus. The main unit internally includes a blood pressure calculation module, a limb pressure application module, a limb pressure sensing module, a blood flow detection control module, and a blood flow detection sensing module.

The limb pressure application module is configured to apply a pressure to or release a pressure from the limb via the bladder-structured cuff. The bladder-structured cuff can encircle the arm and is used to apply a pressure to the arm, to compress the blood vessels in the arm, and occlude blood flow. Specifically, the limb pressure application module includes a first air pump 31, a first inflatable cuff 1, a first rapid exhaust valve 33, and a slow exhaust valve 32. The first air pump 31 is used to inflate the first inflatable cuff 1, while the first rapid exhaust valve 33 and the slow exhaust valve 32 are used for deflation, thereby increasing or reducing the pressure on the limb.

The limb pressure sensing module is used to sense an air pressure signal and collect a pulse wave signal from the bladder-structured cuff. The limb pressure sensing module is a first air pressure sensor 11, configured to sense the air pressure signal and collect the pulse wave signal from the first inflatable cuff 1.

The blood flow detection apparatus is located downstream of the arterial occlusion point at which the bladder-structured cuff is located, and is configured to detect changes in blood flow within the blood vessels. The blood flow detection apparatus employs a second inflatable cuff 2 inflated with a certain amount of gas. The second inflatable cuff 2 is stably fixed downstream of the arterial occlusion point at which the first inflatable cuff 1 is located, and is configured to detect pulse changes when blood flows within the blood vessels. The volume of the second inflatable cuff 2 may be smaller than that of the first inflatable cuff 1, and is configured to detect the blood flow signal and not intended to completely occlude the artery.

The blood flow detection sensing module is configured to collect, from the blood flow detection apparatus, a signal indicating blood flow changes within the blood vessels in the limb. The blood flow detection sensing module is a second air pressure sensor 21, configured to sense, from the second inflatable cuff 2, an air pressure signal indicating blood flow changes.

The blood flow detection control module is configured to control activation and deactivation of the blood flow detection apparatus. The blood flow detection control module includes a second air pump 51 and a second rapid exhaust valve 53, configured to inflate or rapidly deflate the blood flow detection apparatus (that is, the second inflatable cuff 2), thereby increasing or rapidly reducing the pressure on the limb.

The blood pressure calculation module is configured to calculate the systolic blood pressure and diastolic blood pressure based on signals collected by the limb pressure sensing module and the blood flow detection sensing module. The blood pressure calculation module is a controller 40, and specifically, may be a microcontroller unit (MCU), configured to calculate the systolic blood pressure and diastolic blood pressure based on the signals collected by the limb pressure sensing module (first air pressure sensor 11) and the blood flow detection sensing module.

During the process in which the bladder-structured cuff increases the pressure on the limb via the limb pressure application module, the limb pressure sensing module collects the pulse wave signal sensed by the bladder-structured cuff. When the strength of the pulse wave signal weakens to a certain degree, or when the current air pressure inside the bladder-structured cuff exceeds an systolic blood pressure, or when the blood flow detection sensing module fails to collect the blood flow signal within the blood vessels of the limb, the limb pressure application module stops increasing the pressure and begins slow pressure reduction, and the blood pressure calculation module calculates the diastolic blood pressure based on the oscillometric principle.

During the process in which the bladder-structured cuff releases the pressure from the limb via the limb pressure application module, the blood flow detection sensing module detects the pulse oscillation signal when the blood flow resumes. The blood pressure calculation module takes a current signal collected by the corresponding limb pressure sensing module as the systolic blood pressure. The limb pressure application module controls the bladder-structured cuff to rapidly deflate, completing the measurement.

The blood flow detection apparatus is a photoelectric sensing probe, a second inflatable cuff, a radar probe, a Doppler pulse probe, or a piezoelectric sensing apparatus.

The aforementioned "certain degree" is 30-70% of the maximum strength of the pulse wave signal.

In some embodiments, the blood pressure measurement system includes a pressure application apparatus 30, a first sensing apparatus 10, a second sensing apparatus 20, and a controller. The pressure application apparatus 30 is configured to apply a pressure to the user limb in a controlled manner, such as incrementally or decrementally. The first sensing apparatus 10 is configured to obtain a pressure signal; and the second sensing apparatus 20 is configured to obtain the second target signal. The controller 40 can be programmed to control the working process of the aforementioned apparatuses, and calculate the systolic blood pressure and diastolic blood pressure of the user after obtaining the corresponding signals.

The pressure application apparatus 30 includes a first inflatable cuff 1, a first air pump 31, a slow exhaust valve 32, and a first rapid exhaust valve 33. The first air pump 31 can inflate the first inflatable cuff 1 via an air passage. The slow exhaust valve 32 and the first rapid exhaust valve 33 are independently arranged in the air passage between the first air pump 31 and the first inflatable cuff 1. The first inflatable cuff 1 can encircle the arm and, when inflated internally, compress the blood vessels within the arm to occlude blood flow. Besides applying pressure to the limb, the first inflatable cuff 1 further converts a pulse signal into an air pressure signal. Therefore, the first inflatable cuff 1 is also a part of the first sensing apparatus 10. The first sensing apparatus 10 includes a first air pressure sensor 11, the first inflatable cuff 1, and a first tube connecting the two. The first air pressure sensor 11 can detect an air pressure signal inside the first inflatable cuff 1 via the first tube. The second sensing apparatus 20 includes a second air pressure sensor 21, a second inflatable cuff 2, and a second tube connecting the two. The second air pressure sensor 21 can detect an air pressure signal inside the second inflatable cuff 2 via the second tube. The second inflatable cuff 2 is stably fixed downstream of an arterial occlusion point at which the first inflatable cuff 1 is located, and is configured to detect pulse changes when blood flows within the vessel. The air pressure signal from the second inflatable cuff 2 can reflect the arterial pulsation status.

In addition, a second air pump 51 and a second rapid exhaust valve 53 are also disposed in the main unit 3. The second air pump is configured to inflate the second inflatable cuff, and the second rapid exhaust valve is used to deflate the second inflatable cuff. More specifically, the first inflatable cuff 1 and the second inflatable cuff 2 are arranged side by side. The first inflatable cuff 1 is used to wrap around the upper half of the upper arm; and the second inflatable cuff 2 is used to wrap around the lower half of the upper arm. The first inflatable cuff 1 and the second inflatable cuff 2 can be separate or connected side by side as an integrated unit. The main unit 3 is further provided with a port 8 and a port 9, connecting to the tube of the first inflatable cuff 1 and the tube of the second inflatable cuff 2, respectively.

Besides the inflatable cuffs and air pumps, other mechanical structures may alternatively be used to increase and reduce pressure on the limb. For example, in some embodiments, the pressure application apparatus may be a ring or C-shaped hoop with a variable diameter, which uses a mechanical structure rather than pressurized gas to change the diameter of the hoop. When the diameter of the hoop decreases, the hoop gradually compresses the user limb, equivalent to incrementally increasing the pressure on the user limb. When the hoop diameter increases, the hoop gradually reduces the pressure on the user limb.

The first sensing apparatus 10 and the second sensing apparatus 20 are apparatuses for collecting external signals, and perform signal collection operations based on instructions from the controller 40. In some embodiments, the first sensing apparatus 10 may alternatively be a strain sensor paired with the mechanical pressure application apparatus, and the second sensing apparatus 20 maybe a photoelectric sensing apparatus, a radar sensing apparatus, a Doppler sensing apparatus, or a piezoelectric pulse sensing apparatus.

The controller 40 may be an MCU, including a memory and a processor. The controller 40 can send control signals to the pressure application apparatus 30 via a preset program, controlling the pressure application apparatus 30 to apply or release pressure on the limb as commanded. The controller 40 further perform, based on a preset program, calculations on the signals collected by the first sensing apparatus 10 and the second sensing apparatus 20, to obtain the diastolic blood pressure and systolic blood pressure.

After receiving a user command to initiate the measurement process, the blood pressure measurement system can automatically execute the blood pressure measurement method described in the foregoing embodiments. For example, the blood pressure measurement method using the aforementioned blood pressure measurement system includes the following steps:

In S1, that is, a pressure increase process, a limb pressure application module inflates the bladder-structured cuff at an average pressure increase rate V1. The limb pressure sensing module collects pulse wave signals in the bladder-structured cuff. Meanwhile, the blood flow detection control module controls the activation and deactivation of the blood flow detection apparatus. When active, the blood flow detection apparatus can sense a blood flow state of the blood vessels. The blood flow detection sensing module collects a blood flow signal detected by the blood flow detection apparatus.

During the pressure increase process, both the pulse wave signal from the bladder-structured cuff and the blood flow signal at the blood flow detection apparatus are collected simultaneously. When the blood pressure calculation module detects that the strength of the pulse wave signal in the bladder-structured cuff weakens to half of the maximum strength of the pulse wave signal, or when the blood flow signal at the blood flow detection apparatus completely disappears, a current air pressure signal in the bladder-structured cuff is used as the predicted systolic blood pressure SYS1. Subsequently, the limb pressure application module stops pressure increase, and the blood pressure calculation module calculates a diastolic blood pressure DIA according to the oscillometric method.

In S2, that is, a low pressure reduction process, the bladder-structured cuff slowly deflates via the limb pressure application module at a first average pressure reduction rate V21. Simultaneously, the blood flow detection sensing module collects the blood flow signal from the blood flow detection apparatus in real time. When the blood flow detection sensing module collects an oscillation signal generated as the blood flow resumes, a current value of the air pressure in the bladder-structured cuff is defined as the auscultatory-like systolic blood pressure SYS2. At this point, the slow pressure reduction is stopped.

In S3, that is, a rapid pressure reduction process, the limb pressure application module in the main unit controls the bladder-structured cuff to rapidly deflate at an average rate of V22. The blood flow detection control module controls the blood flow detection apparatus to stop detection, ending the measurement process.

The advantages of the blood pressure measurement method and system according to the embodiments of the present disclosure are described in the following comparative results of data from specific measurement processes in the embodiments and comparative examples.

### Embodiment 1

The blood pressure measurement system shown in FIG. 4 to FIG. 6 is used to measure the blood pressure of a user.

Pressure increase process: The first air pump 31 in the pressure application apparatus 30 inflates the first inflatable cuff 1, increasing the internal pressure. The average pressure increase rate V1 may be 10 mmHg/s. Simultaneously, the second air pump 51 inflates the second inflatable cuff 2 at the same rate until the pressure inside the second inflatable cuff 2 reaches 70 mmHg.

During the inflation of the first inflatable cuff 1, pressure oscillation wave signals are collected from both the first inflatable cuff 1 and the second inflatable cuff 2. The controller 40 receives a series of pressure oscillation wave signals sent by the first sensing apparatus 10, performs statistical calculations to obtain the first target signal, that is, a pressure oscillation wave signal with a largest amplitude. When detecting that the amplitude of the pressure oscillation wave signal from the first inflatable cuff 1 weakens to half of the maximum amplitude, a current static pressure in the first inflatable cuff 1 is obtained and used as the predicted systolic blood pressure SYS1. Based on the predicted systolic blood pressure SYS1, a pressure threshold for stopping pressure increase is set, for example, a pressure value 30 mmHg higher than the predicted systolic blood pressure SYS1. Deflation of the first inflatable cuff 1 is stopped when the pressure inside the first inflatable cuff 1 reaches the pressure threshold. At this point, the pressure applied by the first inflatable cuff 1 on the user limb is maximum. The controller 40 calculates the diastolic blood pressure DIA based on the series of pressure oscillation wave signals according to the blood pressure measurement principle of the oscillometric method. In some embodiments, the controller 40 controls the first air pump 31 to stop inflating the first inflatable cuff 1 when the second sensing apparatus 20 fails to detect the third target signal (that is, when the pressure oscillation wave signal in the second inflatable cuff 2 completely disappears).

Pressure reduction process: The slow exhaust valve 32 is opened to release gas from the first inflatable cuff 1, thereby reducing the internal pressure. The pressure reduction rate may be an average of 4 mmHg/s (that is, the first average pressure reduction rate V21=4 mmHg/s). During pressure reduction, the second air pressure sensing apparatus 21 detects the second target signal in the second inflatable cuff 2 in real time, that is, the first pressure oscillation wave signal. The first pressure oscillation wave signal refers to the first signal in two or more consecutive pressure oscillation wave signals detected for the first time. The static pressure in the first inflatable cuff 1 corresponding to the starting moment of the first pressure oscillation wave signal is obtained, that is, the auscultatory-like systolic blood pressure SYS2, which is output as the systolic blood pressure. Upon confirming detection of the second target signal, the first rapid exhaust valve 33 and the second rapid exhaust valve 53 are opened to deflate the first inflatable cuff 1 and the second inflatable cuff 2. The pressure reduction rate may be an average of 80 mmHg/s (that is, the second average pressure reduction rate V22=80 mmHg/s).

For a test subject A whose blood pressure measurement results are a systolic blood pressure of 130 mmHg and a diastolic blood pressure of 80 mmHg, the predicted systolic blood pressure SYS1 is 130 mmHg, and the maximum pressure in the first inflatable cuff 1 is 160 mmHg. The blood pressure measurement time is calculated as follows:
1. Pressure increase time: The pressure increases from 0 mmHg to 160 mmHg at 10 mmHg/s takes 160/10=16 s.
2. Pressure reduction time:
   Pressure reduction to 125 mmHg at a rate of 4 mmHg/s takes (160-125)/4=8.75 s.
   Pressure reduction to 0 mmHg at a rate of 80 mmHg/s takes 125/80≈1.56 s.
   Therefore, the total pressure reduction time is approximately 16 s+8.75 s+1.56 s≈26 s.

The above process is used to measure the blood pressure of a test subject B. The blood pressure measurement results are the systolic blood pressure of 180 mmHg and the diastolic blood pressure of 80 mmHg. The predicted systolic blood pressure SYS1 is 180 mmHg, the maximum pressure increase is 210 mmHg, and the total measurement time is 32 s.

### Comparative example 1

A blood pressure measurement system adopts the principle of the oscillometric method in the pressure increase process. The pressure increase rate during the pressure increase process is 5 mmHg/s. Inflation stops when the cuff pressure rises to 30 mmHg above the systolic blood pressure, and deflation begins at a pressure reduction rate of 80 mmHg/s.

For the test subject A whose blood pressure measurement results are the systolic blood pressure of 130 mmHg and the diastolic blood pressure of 80 mmHg, the measurement time is 34 s.

For the test subject B whose blood pressure measurement results are the systolic blood pressure of 180 mmHg and the diastolic blood pressure of 80 mmHg, the measurement time is 44 s.

### Comparative example 2

A blood pressure measurement system adopts the principle of the oscillometric method in the pressure reduction process. The pressure increase rate during the pressure increase process is 13 mmHg/s. Inflation stops when the cuff pressure rises to 30 mmHg above the systolic blood pressure, and deflation begins at a pressure reduction rate of 5 mmHg/s.

For the test subject A whose blood pressure measurement results are the systolic blood pressure of 130 mmHg and the diastolic blood pressure of 80 mmHg, the measurement time is 33 s.

For the test subject B whose blood pressure measurement results are the systolic blood pressure of 180 mmHg and the diastolic blood pressure of 80 mmHg, the measurement time is 47 s.

### Comparative example 3

A blood pressure measurement system adopts the principle of the auscultatory-like pulse wave method. The pressure increase rate during the pressure increase process is 13 mmHg/s, and pressure increase stops when the pressure reaches 30 mmHg above the systolic blood pressure. The pressure reduction rate during the pressure reduction process is 4 mmHg/s. During pressure reduction, pressure oscillation wave signals are collected from the human body. After the pressure falls to 20 mmHg below the diastolic blood pressure, a rapid pressure reduction at 80 mmHg/s begins until the pressure reaches zero. The systolic blood pressure and diastolic blood pressure are calculated, and the measurement ends.

For the test subject A whose blood pressure measurement results are the systolic blood pressure of 130 mmHg and the diastolic blood pressure of 80 mmHg, the measurement time is 38 s.

For the test subject B whose blood pressure measurement results are the systolic blood pressure of 180 mmHg and the diastolic blood pressure of 80 mmHg, the measurement time is 54 s.

A comparison of blood pressure measurement time between Embodiment 1 and the three comparative examples is as follows:

| No. | Time (Test subject A) | Time (Test subject B) |
|---|---|---|
| Comparative example 1 | 34 s | 44 s |
| Comparative example 2 | 33 s | 47 s |
| Comparative example 3 | 38 s | 54 s |
| Embodiment 1 | 26 s | 32 s |

As can be seen from the data in the table, as the pressure difference between the systolic blood pressure and the diastolic blood pressure increases, the blood pressure measurement method of the present disclosure requires less measurement time compared to the oscillometric and auscultatory-like methods.

### Clinical accuracy comparison experiment between Embodiment 1 and the three comparative examples:

According to the ISO 81060-2:2018 standard for clinical validation of sphygmomanometers, 85 subjects were tested using auscultatory values of the mercury sphygmomanometer as a reference. The comparison of clinical accuracy is as follows:

| No. | Systolic blood pressure | | Diastolic blood pressure | |
|---|---|---|---|---|
| | Mean value | Standard deviation | Mean value | Standard deviation |
| Comparative example 1 | -4.8 | 6.5 | -0.5 | 5.3 |
| Comparative example 2 | -2.6 | 7.5 | -0.7 | 5.5 |
| Comparative example 3 | -1.1 | 5.5 | -2.6 | 8.4 |
| Embodiment 1 | -1.0 | 5.4 | -0.3 | 5.1 |

As can be seen from the data in the table, this embodiment combines the advantages of the oscillometric and auscultatory-like methods. It not only improves accuracy of both the systolic blood pressure and the diastolic blood pressure in non-invasive blood pressure measurement, but also significantly reduces measurement time, thereby enhancing measurement comfort.

### Embodiment 2

Differing from Embodiment 1, in this embodiment, the second sensing apparatus 20 employs a photoelectric sensing apparatus (using green light as the light source). The second sensing apparatus 20 is fixed downstream of the first inflatable cuff 1 along the blood flow direction of the brachial artery in the upper arm, and is configured to detect blood flow oscillation changes when blood flows within the blood vessels.

Referring to FIG. 7, using a test subject with a blood pressure result of 130/80 mmHg and a heart rate of 60 beats/min as an example, the measurement process is as follows:
Pressure increase process: The first air pump 31 inflates the first inflatable cuff 1 at a pressure increase rate of 8 mmHg/pulse (first average pressure increase rate V11=8 mmHg/pulse). When the air pressure inside the first inflatable cuff 1 reaches 80 mmHg, the first inflatable cuff 1 continues to be inflated at a second average pressure increase rate V12 of 13 mmHg/pulse, causing the limb artery to gradually compress and occlude until it is completely blocked. An overall average pressure increase rate V1 is greater than the first average pressure increase rate V11 but less than the second average pressure increase rate V12.

During pressure increase, the pressure oscillation wave signal from the first inflatable cuff 1 and the blood flow signal from the photoelectric sensing apparatus are collected. When the blood flow oscillation signal from the photoelectric sensing apparatus completely disappears, a current value of the air pressure signal in the first inflatable cuff 1 is used as the predicted systolic blood pressure SYS1. Pressure increase continues for an additional 25 mmHg before stopping. The diastolic blood pressure DIA is calculated according to the oscillometric principle. The "predicted systolic blood pressure" estimated above is calculated as: 3*PP-2*DIA. PP is the static pressure in the first inflatable cuff 1 corresponding to a pressure oscillation wave signal with the largest amplitude, that is, an estimated mean arterial pressure. DIA is the estimated diastolic blood pressure, which is the static pressure in the first inflatable cuff 1, corresponding to a pressure oscillation wave signal whose amplitude equals 70% of the maximum amplitude when the amplitude of the pressure oscillation wave signal in the first inflatable cuff 1 increases.

Pressure reduction process: The slow exhaust valve 32 is opened to deflate the first inflatable cuff 1 at a pressure reduction rate of 4 mmHg/pulse. During pressure reduction, the second target signal is collected via the photoelectric sensing apparatus. The static pressure in the first inflatable cuff 1 corresponding to the starting moment of the obtained second target signal is 130 mmHg, that is, the auscultatory-like systolic blood pressure SYS2 is 130 mmHg. The output systolic blood pressure is obtained by calculating the weighted average of the auscultatory-like systolic blood pressure SYS2 and the predicted systolic blood pressure SYS1: SYS=(2*SYS1+8*SYS2)/10, with a weight ratio of 2:8. Subsequently, the first inflatable cuff 1 is deflated via the first rapid exhaust valve at an average pressure reduction rate of 80 mmHg/pulse.

The relationship between the cuff pressure in the first inflatable cuff 1 and time throughout the entire process is shown in FIG. 7.

For the test subject A with a blood pressure measurement result of 130/80 mmHg, the predicted systolic blood pressure SYS1 is 135 mmHg, the maximum pressure increase is 160 mmHg, the auscultatory-like systolic blood pressure SYS2 is 130 mmHg, the finally calculated and output systolic blood pressure SYS is 131 mmHg, and the finally calculated and output diastolic blood pressure DIA is 80 mmHg. The measurement time is calculated as follows:
1. Pressure increase process: The pressure increases from 0 mmHg to 80 mmHg at a rate of 8 mmHg/pulse, and the pressure increases from 80 mmHg to 160 mmHg at a rate of 13 mmHg/pulse. The pressure increase time is 80/8+(160-80)/13≈16.15 s.
2. After the pressure in the first inflatable cuff 1 reaches 160 mmHg, the pressure reduces to 125 mmHg at a rate of 4 mmHg/pulse. The pressure reduction time is (160-125)/4=8.75 s.
3. Finally, the pressure rapidly reduces at a rate of 80 mmHg/pulse to end the measurement. The rapid pressure reduction time is 125/80≈1.56 s.

Thus, the total blood pressure measurement time is approximately 26 s.

For the test subject B with a blood pressure measurement result of 180/80 mmHg, the predicted systolic blood pressure SYS1 is 185 mmHg, the maximum pressure increase is 210 mmHg, the auscultatory-like systolic blood pressure SYS2 is 180 mmHg, the finally calculated and output systolic blood pressure SYS is 181 mmHg, and the finally calculated and output diastolic blood pressure is 80 mmHg. The measurement time is calculated as follows:
1. Pressure increase process: The pressure increases from 0 mmHg to 80 mmHg at a rate of 8 mmHg/pulse, and the pressure increases from 80 mmHg to 210 mmHg at a rate of 13 mmHg/pulse. The pressure increase time is 80/8+(210-80)/13=20 s.
2. After the pressure in the first inflatable cuff 1 reaches 210 mmHg, the pressure reduces to 175 mmHg at a rate of 4 mmHg/pulse. The pressure reduction time is (210-175)/4=8.75 s.
3. Finally, the pressure rapidly reduces at a rate of 80 mmHg/pulse to end the measurement. The rapid pressure reduction time is 175/80≈2.19 s.

Thus, the total blood pressure measurement time is approximately 31 s.

A comparison of overall measurement time between Embodiment 2 and the three comparative examples is as follows:

| No. | Time (Test subject A) | Time (Test subject B) |
|---|---|---|
| Comparative example 1 | 34 s | 44 s |
| Comparative example 2 | 33 s | 47 s |
| Comparative example 3 | 38 s | 54 s |
| Embodiment 2 | 26 s | 31 s |

As can be seen from the data in the table, as the pressure difference between the systolic blood pressure and the diastolic blood pressure increases, the reduction in measurement time achieved by the present disclosure is more significant compared to the oscillometric and auscultatory-like methods.

### Clinical accuracy comparison experiment between Embodiment 2 and the three comparative examples:

According to the ISO 81060-2:2018 standard for clinical validation of sphygmomanometers, 85 subjects were tested using auscultatory values of the mercury sphygmomanometer as a reference. The comparison of clinical accuracy is as follows:

| | Measurement type | Systolic blood pressure | | Diastolic blood pressure | |
|---|---|---|---|---|---|
| | | Mean value | Standard deviation | Mean value | Standard deviation |
| Comparative example 1 | An oscillometric sphygmomanometer for measurement in a pressure increase process | -4.8 | 6.5 | -0.5 | 5.3 |
| Comparative example 2 | An oscillometric sphygmomanometer for measurement in a pressure reduction process | -2.6 | 7.5 | -0.7 | 5.5 |
| Comparative example 3 | A sphygmomanometer using the auscultatory-like pulse wave method | -1.1 | 5.5 | -2.6 | 8.4 |
| Embodiment 2 | A sphygmomanometer of this embodiment | -0.5 | 5.6 | -0.5 | 4.9 |

As can be seen from the data in the table, this embodiment combines the advantages of the oscillometric and auscultatory-like methods. It not only improves accuracy of both the systolic blood pressure and the diastolic blood pressure in non-invasive blood pressure measurement, but also significantly reduces measurement time, thereby enhancing measurement comfort.

### Embodiment 3

In this embodiment:
The bladder-structured cuff is a first inflatable cuff 1 capable of encircling the arm, and is used to apply a pressure to the upper arm at an average rate V1, to compress the blood vessels within the arm, and occlude blood flow.

The second sensing apparatus 20 employs a Doppler pulse probe or a radar probe, positioned beneath the first inflatable cuff 1, to detect blood flow oscillation changes when blood flows within the blood vessels. Differing from Embodiment 1, in this embodiment, the blood flow detection control module is a photoelectric drive circuit; and the blood flow detection sensing module is a photoelectric collection circuit, configured to collect blood flow oscillation change signals from the photoelectric sensing apparatus when blood flows.

The blood flow detection control module is a Doppler drive circuit or a radar drive circuit. The blood flow detection sensing module is a Doppler signal collection circuit or a radar signal collection circuit, configured to collect the pulse wave oscillation signal from the Doppler pulse probe or the radar probe when blood flows.

For the test subject A with a blood pressure measurement result of 130/80 mmHg, the measurement method is as follows:
In S1, that is, the pressure increase process, the first air pump 31 in the pressure application apparatus 30 inflates the first inflatable cuff 1 at an average rate V1 of 8 mmHg/s, causing the limb artery to gradually compress and occlude until it is completely blocked.

Simultaneously, the pressure oscillation wave signal from the first inflatable cuff 1 and the blood flow signal from the Doppler pulse probe or the radar probe are collected. When the strength of the pressure oscillation wave signal from the first inflatable cuff 1 weakens to half of the maximum strength of the pressure oscillation wave signal, or when the blood flow oscillation change signal from the Doppler pulse probe or radar probe completely disappears, a current value of the air pressure signal in the first inflatable cuff 1 is used as the predicted systolic blood pressure SYS1. Pressure increase continues for an additional 20 mmHg before stopping. At this point, the maximum pressure increase is approximately 160 mmHg. The diastolic blood pressure DIA is calculated according to the oscillometric principle.

In S2, that is, the slow pressure reduction process, the first inflatable cuff 1 deflates via the slow exhaust valve 32 inside the main unit 3 at an average rate V21 of 8 mmHg/s. During this period, the Doppler signal collection circuit or radar signal collection circuit collects the pulse oscillation signal from the Doppler pulse probe or radar probe in real time. When the Doppler pulse probe or radar probe consecutively detects two or more blood flow signals, the air pressure in the first inflatable cuff 1 corresponding to the starting moment of the first blood flow signal is used as the auscultatory-like systolic blood pressure SYS2. The air pressure slowly decreases to approximately 125 mmHg since then.

In S3, that is, the rapid pressure reduction process, the first inflatable cuff 1 deflates via the first rapid exhaust valve 33 at an average rate V22 of 25 mmHg/s, to end the measurement.

For a test subject with a blood pressure measurement result of 130/80 mmHg, the predicted systolic blood pressure SYS1 is 140 mmHg, the maximum pressure increase is 160 mmHg, the finally calculated and output systolic blood pressure SYS is 130 mmHg, and the finally calculated and output diastolic blood pressure is 80 mmHg. The measurement time is calculated as follows:
In the pressure increase process, the pressure increases from 0 mmHg to 160 mmHg at a rate of 8 mmHg/s. Once the pressure in the first inflatable cuff 1 reaches 160 mmHg, the pressure reduces to 125 mmHg at a rate of 8 mmHg/s. Finally, the pressure rapidly reduces at a rate of 25 mmHg/s, to end the measurement. The total time for blood pressure measurement is: 160/8+(160-125)/8+125/25≈29 s.

For a test subject with a blood pressure measurement result of 180/80 mmHg, the predicted systolic blood pressure SYS1 is 190 mmHg, the maximum pressure increase is 210 mmHg, the finally calculated and output systolic blood pressure SYS is 180 mmHg, and the finally calculated and output diastolic blood pressure is 80 mmHg. The measurement time is calculated as follows:
In the pressure increase process, the pressure increases from 0 mmHg to 210 mmHg at a rate of 8 mmHg/s. Once the pressure in the first inflatable cuff 1 reaches 210 mmHg, the pressure reduces to 175 mmHg at a rate of 8 mmHg/s. Finally, the pressure rapidly reduces at a rate of 25 mmHg/s, to end the measurement. The total time for blood pressure measurement is: 210/8+(210-175)/8+175/25≈38 s.

Using the test subjects with blood pressures of 130/80 mmHg and 180/80 mmHg as examples, Embodiment 3 demonstrates a significant reduction in measurement time compared to the three comparative examples, thereby improving measurement comfort.

### Embodiment 4

In this embodiment:
The bladder-structured cuff is an inflatable wristband capable of encircling the wrist, and is used to apply a pressure to the wrist at an average rate V1, to compress the blood vessels within the wrist, and occlude blood flow.

The second sensing apparatus 20 employs a photoelectric sensing apparatus, applied to a finger. The light source uses red or infrared light to detect blood flow oscillation changes when blood flows within the blood vessels.

The pressure application apparatus 30 includes a first air pump 31, a first rapid exhaust valve 33, the inflatable wristband, and a slow exhaust valve 32, and is configured to inflate or deflate the inflatable wristband, thereby increasing or reducing pressure on the limb. The limb pressure sensing module is the first air pressure sensor 11, configured to sense an air pressure signal and collect a pressure oscillation wave signal from the inflatable wristband. This design is consistent with that in Embodiment 1.

The blood flow detection control module is a photoelectric drive circuit. The blood flow detection sensing module is a photoelectric collection circuit, configured to collect blood flow oscillation change signals from the photoelectric sensing apparatus when blood flows.

Taking the test subject A with the blood pressure result of 130/80 mmHg as an example, the measurement method is as follows:
In S1, that is, the pressure increase process, the first air pump 31 in the pressure application apparatus 30 inflates the inflatable wristband at an average rate V11 of 10 mmHg/s (the average rate for the low-pressure phase of V1). When the air pressure inside the inflatable wristband reaches 80 mmHg, the inflatable wristband continues to be inflated at an average rate V12 of 20 mmHg/s (the average rate for the high-pressure phase of V1). This causes the wrist artery to gradually compress and occlude until it is completely blocked.

Simultaneously, the pressure oscillation wave signal from the inflatable wristband and the blood flow signal from the photoelectric sensing apparatus are collected. When the strength of the pressure oscillation wave signal from the inflatable wristband weakens to half of the maximum strength of the pressure oscillation wave signal, or when the blood flow oscillation change signal from the photoelectric sensing apparatus completely disappears, a current value of the air pressure signal in the inflatable wristband is used as the predicted systolic blood pressure SYS1. Pressure increase continues for an additional 30 mmHg before stopping. At this point, the maximum pressure increase is approximately 160 mmHg. The diastolic blood pressure DIA is calculated according to the oscillometric principle.

In S2, that is, the slow pressure reduction process, the inflatable wristband slowly deflates via the slow exhaust valve 32 inside the main unit 3 at an average rate V21 of 5 mmHg/pulse. During this period, the photoelectric collection circuit collects the blood flow oscillation change signals from the photoelectric sensing apparatus in real time. When the photoelectric sensing apparatus consecutively detects two or more blood flow signals, the air pressure in the inflatable wristband corresponding to the starting moment of the first blood flow signal is used as the systolic blood pressure SYS. The air pressure slowly decreases to approximately 125 mmHg since then.

In S3, that is, the rapid pressure reduction process, the inflatable wristband rapidly deflates via the first rapid exhaust valve 31 at an average rate V22 of 50 mmHg/s, to end the measurement.

For a test subject with a blood pressure measurement result of 130/80 mmHg, the predicted systolic blood pressure SYS1 is 130 mmHg, the maximum pressure increase is 160 mmHg, the finally calculated and output systolic blood pressure SYS is 130 mmHg, and the finally calculated and output diastolic blood pressure is 80 mmHg. The measurement time is calculated as follows:
In the pressure increase process, the pressure increases from 0 mmHg to 80 mmHg at a rate of 10 mmHg/s and the pressure increases from 80 mmHg to 160 mmHg at a rate of 20 mmHg/s. Once the pressure in the inflatable wristband reaches 160 mmHg, the pressure reduces to 125 mmHg at a rate of 5 mmHg/s. Finally, the pressure rapidly reduces at a rate of 50 mmHg/s, to end the measurement. The total time for blood pressure measurement is: 80/10+(160-80)/20+(160-125)/5+125/50≈22 s.

For a test subject with a blood pressure measurement result of 180/80 mmHg, the predicted systolic blood pressure SYS1 is 180 mmHg, the maximum pressure increase is 210 mmHg, the finally calculated and output systolic blood pressure SYS is 180 mmHg, and the finally calculated and output diastolic blood pressure is 80 mmHg. The measurement time is calculated as follows:
In the pressure increase process, the pressure increases from 0 mmHg to 100 mmHg at a rate of 10 mmHg/s and the pressure increases from 100 mmHg to 210 mmHg at a rate of 20 mmHg/s. Once the pressure in the inflatable wristband reaches 210 mmHg, the pressure reduces to 175 mmHg at a rate of 5 mmHg/s. Finally, the pressure rapidly reduces at a rate of 50 mmHg/s, to end the measurement. The total time for blood pressure measurement is: 100/10+(210-100)/20+(210-175)/5+175/50≈26 s.

Using the test subjects with blood pressures of 130/80 mmHg and 180/80 mmHg as examples, Embodiment 4 demonstrates a significant reduction in measurement time compared to the three comparative examples, thereby improving measurement comfort.

### Embodiment 5

In this embodiment:
The bladder-structured cuff is a first inflatable cuff 1 capable of encircling the arm, and is used to apply a pressure to the upper arm at an average rate V1, to compress the blood vessels within the arm, and occlude blood flow.

The second sensing apparatus 20 employs a piezoelectric sensing apparatus, positioned downstream of the first inflatable cuff 1 along the arterial blood flow direction in the arm, and configured to detect blood flow oscillation changes when blood flows within the blood vessels.

The pressure application apparatus 30 includes a first air pump 31, a first rapid exhaust valve 33, and a slow exhaust valve 32, and is configured to inflate or deflate the first inflatable cuff 1, thereby increasing or decreasing the pressure on the arm. The limb pressure sensing module is the first air pressure sensor 11, configured to sense an air pressure signal and collect a pressure oscillation wave signal from the first inflatable cuff 1. This design is consistent with that in Embodiment 1.

The blood flow detection control module is a piezoelectric drive circuit. The blood flow detection sensing module is a piezoelectric collection circuit, configured to collect blood flow oscillation change signals from the piezoelectric sensing apparatus when blood flows.

Taking a measurement of a test subject with a systolic blood pressure of 130 mmHg and a diastolic blood pressure of 80 mmHg as an example, the measurement method is as follows:
In S1, that is, the pressure increase process, the first air pump in the pressure application apparatus inflates the first inflatable cuff 1 at an average rate V11 of 15 mmHg/s (the average rate for the low-pressure phase of V1). When the air pressure inside the first inflatable cuff 1 reaches 80 mmHg, the first inflatable cuff 1 continues to be inflated at an average rate V12 of 25 mmHg/s (the average rate for the high-pressure phase of V1). This causes the limb artery to gradually compress and occlude until it is completely blocked.

Simultaneously, the pressure oscillation wave signal from the first inflatable cuff 1 and the blood flow signal from the piezoelectric sensing apparatus are collected. When the strength of the pressure oscillation wave signal from the first inflatable cuff 1 weakens to half of the maximum strength of the pressure oscillation wave signal, or the blood flow oscillation signal from the piezoelectric sensing apparatus completely disappears, a current value of the air pressure signal in the first inflatable cuff 1 is used as the predicted systolic blood pressure SYS1. Pressure increase continues for an additional 25 mmHg before stopping. At this point, the maximum pressure increase is approximately 160 mmHg. The diastolic blood pressure DIA is calculated according to the oscillometric principle.

In S2, that is, the slow pressure reduction process, the first inflatable cuff 1 deflates via the slow exhaust valve inside the main unit at an average rate V21 of 2 mmHg/s. During this period, the piezoelectric collection circuit collects the blood flow oscillation signal from the piezoelectric sensing apparatus in real time. When the piezoelectric sensing apparatus consecutively detects two or more blood flow signals, the air pressure in the first inflatable cuff 1 corresponding to the starting moment of the first blood flow signal is used as the predicted systolic blood pressure SYS2. The air pressure slowly decreases to approximately 125 mmHg since then.

In S3, that is, the rapid pressure reduction process, the first inflatable cuff 1 deflates via the rapid exhaust valve 1 at an average rate V22 of 100 mmHg/s, to end the measurement.

For a test subject with a blood pressure measurement result of 130/80 mmHg, the predicted systolic blood pressure SYS1 is 135 mmHg, the maximum pressure increase is 160 mmHg, the finally calculated and output systolic blood pressure SYS is 130 mmHg, and the finally calculated and output diastolic blood pressure is 80 mmHg. The measurement time is calculated as follows:
In the pressure increase process, the pressure increases from 0 mmHg to 80 mmHg at a rate of 15 mmHg/s and the pressure increases from 80 mmHg to 160 mmHg at a rate of 25 mmHg/s. Once the pressure in the first inflatable cuff 1 reaches 160 mmHg, the pressure reduces to 125 mmHg at a rate of 2 mmHg/s. Finally, the pressure rapidly reduces at a rate of 100 mmHg/s, to end the measurement. The total time for blood pressure measurement is: 80/15+(160-80)/25+(160-125)/2+125/100≈27 s.

For a test subject with a blood pressure measurement result of 180/80 mmHg, the predicted systolic blood pressure SYS1 is 185 mmHg, the maximum pressure increase is 210 mmHg, the finally calculated and output systolic blood pressure SYS is 180 mmHg, and the finally calculated and output diastolic blood pressure is 80 mmHg. The measurement time is calculated as follows:
In the pressure increase process, the pressure increases from 0 mmHg to 80 mmHg at a rate of 15 mmHg/pulse and the pressure increases from 80 mmHg to 210 mmHg at a rate of 25 mmHg/pulse. Once the pressure in the first inflatable cuff 1 reaches 210 mmHg, the pressure reduces to 175 mmHg at a rate of 2 mmHg/s. Finally, the pressure rapidly reduces at a rate of 100 mmHg/s, to end the measurement. The total time for blood pressure measurement is: 80/15+(210-80)/25+(210-175)/2+175/100≈30 s.

Using the test subjects with blood pressures of 130/80 mmHg and 180/80 mmHg as examples, Embodiment 5 demonstrates a significant reduction in measurement time compared to the three comparative examples, thereby improving measurement comfort.

### Embodiment 6

In this embodiment:
A mechanical band and a piezoelectric sensing apparatus are used to replace the bladder-structured cuff. The piezoelectric sensing apparatus is positioned between the mechanical band and the upper arm. The mechanical band is wrapped around and fastened to the upper arm, and is capable of applying a pressure to the upper arm at an average rate V1, to compress the blood vessels within the arm, and occlude blood flow.

The second sensing apparatus 20 employs a photoelectric sensing apparatus, stably fixed downstream of the mechanical band along the arterial blood flow direction in the arm, and configured to detect blood flow oscillation changes when blood flows within the blood vessels.

The pressure application apparatus 30 includes a servo driver (not shown in the figure) and a servo motor (not shown in the figure), configured to control the mechanical band in increasing or reducing the pressure on the arm. The limb pressure sensing module is a piezoelectric sensor, configured to collect pressure signals and pressure oscillation wave signals from the piezoelectric sensing apparatus.

The blood flow detection control module is a photoelectric drive circuit. The blood flow detection sensing module is a photoelectric collection circuit, configured to collect blood flow oscillation change signals from the photoelectric sensing apparatus.

Taking a blood pressure measurement of a test subject with a systolic blood pressure of 130 mmHg and a diastolic blood pressure of 80 mmHg as an example, the measurement method is as follows:
In S1, that is, a pressure increase process, the servo driver and servo motor control the mechanical band to apply a pressure to the arm at an average rate V11 of 15 mmHg/s (the average rate for the low-pressure phase of V1). When the pressure sensed by the piezoelectric sensing apparatus reaches 80 mmHg, the pressure on the arm continues to increase at an average rate V12 of 35 mmHg/s (the average rate for the high-pressure phase of V1). This causes the limb artery to gradually compress and occlude until it is completely blocked.

Simultaneously, the pressure oscillation wave signal sensed by the piezoelectric sensing apparatus and the blood flow signal from the photoelectric sensing apparatus are collected. When the strength of the pressure oscillation wave signal sensed by the piezoelectric sensing apparatus weakens to half of the maximum strength of the pressure oscillation wave signal, or when the blood flow oscillation change signal from the photoelectric sensing apparatus completely disappears, a current value sensed by the piezoelectric sensing apparatus is used as the predicted systolic blood pressure SYS1. Pressure increase continues for an additional 25 mmHg before stopping. At this point, the maximum pressure increase is approximately 160 mmHg. The diastolic blood pressure DIA is calculated according to the oscillometric principle.

In S2, that is, a slow pressure reduction process, the mechanical band slowly releases the pressure at an average rate V21 of 1 mmHg/s. During this period, the photoelectric collection circuit collects the blood flow oscillation change signals from the photoelectric sensing apparatus in real time. When the photoelectric sensing apparatus consecutively detects two or more blood flow signals, the air pressure sensed by the piezoelectric sensing apparatus and corresponding to the starting moment of the first blood flow signal is used as the auscultatory-like systolic blood pressure SYS2. By this point, the applied pressure has slowly decreased to approximately 125 mmHg.

In S3, that is, a rapid pressure reduction process, the mechanical band rapidly releases the pressure at an average rate V22 of 120 mmHg/s, to end the measurement.

For a test subject with a blood pressure measurement result of 130/80 mmHg, the predicted systolic blood pressure SYS1 is 135 mmHg, the maximum pressure increase is 160 mmHg, the finally calculated and output systolic blood pressure SYS is 130 mmHg, and the finally calculated and output diastolic blood pressure is 80 mmHg. The measurement time is calculated as follows:
In the pressure increase process, the pressure increases from 0 mmHg to 100 mmHg at a rate of 15 mmHg/s, and the pressure increases from 100 mmHg to 160 mmHg at a rate of 35 mmHg/pulse. Once the pressure of the piezoelectric sensing apparatus reaches 160 mmHg, the pressure reduces to 125 mmHg at a rate of 2 mmHg/s. Finally, the pressure rapidly reduces at a rate of 120 mmHg/s, to end the measurement. The total time for blood pressure measurement is: 100/15+(160-100)/35+(160-125)/2+125/120≈27 s.

For a test subject with a blood pressure measurement result of 180/80 mmHg, the predicted systolic blood pressure SYS1 is 185 mmHg, the maximum pressure increase is 210 mmHg, the systolic blood pressure SYS is 180 mmHg, and the diastolic blood pressure is 80 mmHg. The measurement time is calculated as follows:
In the pressure increase process, the pressure increases from 0 mmHg to 100 mmHg at a rate of 15 mmHg/pulse and the pressure increases from 100 mmHg to 210 mmHg at a rate of 35 mmHg/pulse. Once the pressure of the piezoelectric sensing apparatus reaches 210 mmHg, the pressure reduces to 175 mmHg at a rate of 2 mmHg/s. Finally, the band is released at a rate of 120 mmHg/s, to end the measurement. The total time for blood pressure measurement is: 100/15+(210-100)/35+(210-175)/2+175/120≈29 s.

Using the test subjects with blood pressures of 130/80 mmHg and 180/80 mmHg as examples, Embodiment 6 demonstrates a significant reduction in measurement time compared to the three comparative examples, thereby improving measurement comfort.

The above merely describes specific implementations of the present disclosure, but the protection scope of the present disclosure is not limited thereto. Any person skilled in the art can easily conceive various equivalent modifications or replacements within the technical scope of the present disclosure, and these modifications or replacements shall fall within the protection scope of the present disclosure. Therefore, the protection scope of the present disclosure shall be subject to the protection scope of the claims.

## Claims

1. A blood pressure measurement method, comprising:
incrementally applying a pressure to a user limb to block a target artery;
obtaining, during pressure application to the user limb, at least a plurality of pressure oscillation wave signals generated by pulsations of the target artery;
obtaining a first target signal based on the plurality of pressure oscillation wave signals, and obtaining a diastolic blood pressure based on the first target signal;
decrementally applying the pressure to the user limb;
detecting a second target signal during decremental pressure application to the user limb, and obtaining the pressure applied to the user limb when the second target signal is detected, wherein the second target signal is used to represent a state transition of the target artery from an occluded state to a perfused state; and
obtaining a systolic blood pressure based on at least the pressure applied to the user limb when the second target signal is detected.

2. The blood pressure measurement method according to claim 1, wherein
decrementally applying the pressure to the user limb comprises: reducing the pressure at a first average pressure reduction rate, and subsequently reducing the pressure at a second average pressure reduction rate; and
detecting the second target signal during pressure reduction comprises: detecting the second target signal during pressure reduction at the first average pressure reduction rate, wherein
the second average pressure reduction rate is greater than the first average pressure reduction rate.

3. The blood pressure measurement method according to claim 2, comprising: switching to reducing the pressure at the second average pressure reduction rate in response to confirming detection of the second target signal.

4. The blood pressure measurement method according to claim 1, wherein
incrementally applying the pressure to the user limb comprises: first applying the pressure at a first average pressure increase rate and subsequently applying the pressure at a second average pressure increase rate, wherein the first average pressure increase rate is less than the second average pressure increase rate.

5. The blood pressure measurement method according to claim 1, comprising: detecting a third target signal during pressure application, and stopping applying the pressure in response to failing to detect the third target signal; or stopping applying the pressure in response to reaching a preset pressure threshold, wherein the preset pressure threshold is set based on the first target signal.

6. The blood pressure measurement method according to claim 5, wherein the third target signal is a fluctuation signal obtained from downstream of the target artery to which the pressure is applied during incremental pressure application to the user limb, the fluctuation signal is an air pressure signal, a photoelectric signal, an electromagnetic wave signal, an ultrasonic signal, or a piezoelectric signal, and the fluctuation signal reflects changes in blood flow.

7. The blood pressure measurement method according to claim 1, wherein the first target signal is a preset pressure oscillation wave signal obtained based on the plurality of pressure oscillation wave signals, and/or the second target signal is a first fluctuation signal detected during decremental pressure application to the user limb, the fluctuation signal is an air pressure signal, a photoelectric signal, an electromagnetic wave signal, an ultrasonic signal, or a piezoelectric signal, and the fluctuation signal reflects changes in blood flow.

8. The blood pressure measurement method according to claim 7, wherein the first fluctuation signal is a first fluctuation signal of at least two consecutive fluctuation signals detected for a first time.

9. The blood pressure measurement method according to claim 1, further comprising: calculating a predicted systolic blood pressure based on the first target signal, and calculating the systolic blood pressure based on the predicted systolic blood pressure and the pressure applied to the user limb when the second target signal is detected.

10. The blood pressure measurement method according to claim 1, wherein calculating the systolic blood pressure based on at least the second target signal comprises: based on a moment of detecting the second target signal, obtaining the pressure applied to the user limb at the moment.

11. The blood pressure measurement method according to claim 1, wherein the second target signal is detected downstream of a position at which the target artery is occluded.

12. A blood pressure measurement system, comprising:
a pressure application apparatus configured to apply a pressure to a user limb;
a first sensing apparatus configured to obtain pressure signals;
a second sensing apparatus configured to obtain a second target signal; and
a controller programmed to:
control the pressure application apparatus to incrementally apply the pressure to the user limb to block a target artery;
control the first sensing apparatus to obtain at least a plurality of pressure oscillation wave signals generated by pulsations of the target artery during pressure application to the user limb;
calculate a first target signal based on the plurality of pressure oscillation wave signals, and obtain a diastolic blood pressure based on the first target signal;
control the pressure application apparatus to decrementally apply the pressure to the user limb;
control the second sensing apparatus to detect a second target signal during decremental pressure application to the user limb, and obtain, from the first sensing apparatus, the pressure applied to the user limb when the second target signal is detected, wherein the second target signal is used to represent a state transition of the target artery in the user limb from an occluded state to a perfused state; and
obtain a systolic blood pressure based on at least the pressure applied to the user limb when the second target signal is detected.

13. The blood pressure measurement system according to claim 12, wherein the controller is programmed to:
control the pressure application apparatus to reduce the pressure at a first average pressure reduction rate, and subsequently reduce the pressure at a second average pressure reduction rate during the decremental pressure application to the user limb; and
control the second sensing apparatus to detect, when detecting the second target signal during pressure reduction, the second target signal during pressure reduction at the first average pressure reduction rate, wherein
the second average pressure reduction rate is greater than the first average pressure reduction rate.

14. The blood pressure measurement system according to claim 13, wherein the controller is programmed to control the pressure application apparatus to switch to reducing the pressure at the second average pressure reduction rate in response to confirming detection of the second target signal.

15. The blood pressure measurement system according to claim 12, wherein the controller is programmed to:
control the pressure application apparatus to apply the pressure at a first average pressure increase rate, and subsequently apply the pressure at a second average pressure increase rate during incremental pressure application to the user limb, wherein
the first average pressure increase rate is less than the second average pressure increase rate.

16. The blood pressure measurement system according to claim 12, wherein the controller is programmed to control the second sensing apparatus to detect a third target signal when the pressure application apparatus applies the pressure, and control the pressure application apparatus to:
stop applying the pressure in response to failing to detect the third target signal; or stop applying the pressure in response to reaching a preset pressure threshold, wherein the preset pressure threshold is set based on the first target signal.

17. The blood pressure measurement system according to claim 16, wherein the third target signal is a fluctuation signal obtained from downstream of the target artery to which the pressure is applied during incremental pressure application to the user limb, the fluctuation signal is an air pressure signal, a photoelectric signal, an electromagnetic wave signal, an ultrasonic signal, or a piezoelectric signal, and the fluctuation signal reflects changes in blood flow.

18. The blood pressure measurement system according to claim 12, wherein the first target signal is a preset pressure oscillation wave signal obtained based on the plurality of pressure oscillation wave signals, and/or the second target signal is a first fluctuation signal detected during reduction of the pressure applied to the user limb, the fluctuation signal is an air pressure signal, a photoelectric signal, an electromagnetic wave signal, an ultrasonic signal, or a piezoelectric signal, and the fluctuation signal reflects changes in blood flow.

19. The blood pressure measurement system according to claim 18, wherein the first fluctuation signal is a first fluctuation signal of at least two consecutive fluctuation signals detected for a first time.

20. The blood pressure measurement system according to claim 12, wherein the controller further calculates a predicted systolic blood pressure based on the first target signal, and calculates the systolic blood pressure based on the predicted systolic blood pressure and the pressure applied to the user limb when the second target signal is detected.

21. The blood pressure measurement system according to claim 12, wherein a process in which the controller calculates the systolic blood pressure based on at least the second target signal comprises: based on a moment of detecting the second target signal, obtaining the pressure applied to the user limb at the moment.

22. The blood pressure measurement system according to claim 12, wherein the second sensing apparatus is configured to detect the second target signal downstream of a position at which the target artery is occluded.
